**Europäisches Patentamt**

(19) **European Patent Office**

**Office européen des brevets**

(11) Numéro de publication: **0 101 337**
**B1**

(12) # FASCICULE DE BREVET EUROPEEN

(45) Date de publication du fascicule du brevet:
21.08.85

(51) Int. Cl.⁴: **C 07 C 19/02**, C 07 C 17/10

(21) Numéro de dépôt: **83401314.6**

(22) Date de dépôt: **24.06.83**

(54) **Procédé de fabrication du chlorure de méthylène.**

(30) Priorité: **06.07.82 FR 8211811**

(43) Date de publication de la demande:
**22.02.84 Bulletin 84/8**

(45) Mention de la délivrance du brevet:
**21.08.85 Bulletin 85/34**

(84) Etats contractants désignés:
**BE DE FR GB IT NL SE**

(56) Documents cités:
**CH - A - 360 053**
**FR - A - 1 108 804**

(73) Titulaire: **ATOCHEM, 12/16, allée des Vosges,**
**F-92400 Courbevoie (FR)**

(72) Inventeur: **Olah, George Andrew, 2252 Gloaming Way,**
**Beverly Hills California 90210 (US)**
Inventeur: **Gurtner, Bernard, 5 Boulevard Auguste**
**Sembat, F-38000 Grenoble (FR)**
Inventeur: **Gorny, Bernard, Le Cert Champ-sur-Drac,**
**F-38560 Jarrie (FR)**

BUNDESDRUCKEREI BERLIN

## Description

L'invention est relative à un procédé de fabrication de chlorure de méthylène basé sur une chloration sélective de chlorure de méthyle en chlorure de méthylène par un mécanisme ionique.

Le chlorure de méthylène est un solvant largement utilisé au niveau industriel, et ses applications sont nombreuses: agent de dégraissage, solvant de peinture, aérosols, agent d'extraction, etc.

De nombreuses publications décrivent la synthèse de ce chlorométhane par chloration de chlorure de méthyle, mais toutes ces préparations mettent en oeuvre un mécanisme radicalaire: ces radicaux libres sont formés soit par apport thermique, soit par irradiation photochimique ou encore par des initiateurs chimiques; ces procédés ne permettent pas de synthétiser un chlorométhane donné comme, par exemple, le chlorure de méthylène. Par chloration thermique du chlorure de méthyle à 440°C, suivant un mécanisme radicalaire, on obtient un mélange gazeux constitué de chlorure de méthylène, de chloroforme et de tétrachlorure de carbone. De plus la chloration thermique, comme cela a été illustré plus haut, nécessite l'emploi de températures élevées avec tous les ennuis qui peuvent en découler.

C'est pourquoi il a, par exemple, été proposé de pallier ces inconvénients par l'emploi d'un très fort excès de chlorure de méthyle par rapport au chlore mis en oeuvre. Ainsi, dans le brevet européen 5296, est décrit un procédé de chloration de chlorure de méthyle en chlorure de méthylène, les radicaux libres étant fournis au milieu réactionnel par un initiateur chimique; pour ce faire, le rapport molaire $CH_3Cl/Cl_2$ utilisé pour la synthèse est compris entre 10 et 2. Mais cette façon d'opérer nécessite des recyclages répétés de chlorure de méthyle et conduit donc à des consommations d'énergie importantes pour assurer ce recyclage.

D'autres procédés décrivant encore la transformation ultérieure du chloroforme ou du tétrachlorure de carbone indésirables en chlorure de méthylène par hydrogénation; mais cette façon d'opérer nécessite une phase supplémentaire induisant des coûts complémentaires d'investissement et de fabrication peu souhaitables.

Il a été découvert qu'il est possible d'obtenir sélectivement du chlorure de méthylène par chloration du chlorure de méthyle, et ce, sans se trouver confrontés aux inconvénients précités. Dans le procédé selon l'invention, il est possible d'obtenir du chlorure de méthylène avec une sélectivité de 80 à 98% pour un taux de conversion pondéral du réactif de départ, le chlorure de méthyle, pouvant atteindre au moins 40%, en utilisant un catalyseur permettant un mécanisme de chloration ionique, et avec un rapport molaire $CH_3Cl/Cl_2$ compris entre 3 et 0,25, et de préférence entre 2 et 0,3.

Les catalyseurs métalliques utilisables pour réaliser cette chloration ionique font partie des groupes III, IV, V, VI et VIII de la table périodique. Les catalyseurs sont mis en oeuvre sous la forme habituelle d'utilisation de ces types de catalyseurs telle que métallique, oxyde, sel et autres; dans le cas de la présente invention, on préfère les mettre en oeuvre sous forme métallique ou encore d'halogénure ou d'oxyhalogénure et plus particulièrement de chlorure ou d'oxychlorure. Ils peuvent être employés tel quel ou déposés sur un support. A titre d'exemple, on peut citer les catalyseurs à base de fer, zirconium, tungstène, antimoine, gallium, aluminium, zinc, ou leurs mélanges ou encore platine, palladium et autres déposés éventuellement sur alumine, zircone, zircon, silice, carbone etc.

En opérant avec ces catalyseurs, à des températures comprises entre 150 et 350°C, mais de préférence entre 170 et 300°C, avec des rapports molaires $CH_3Cl/Cl_2$ compris entre 2 et 0,25 et de préférence entre 1,5 et 0,3, on obtient du chlorure de méthylène avec une grande sélectivité, le chloroforme étant minimisé et le tétrachlorure de carbone pratiquement absent. Les exemples ci-après illustrent l'invention.

### Exemple 1

A une solution agitée de 20 g de $FeCl_3$ dans 100 ml d'eau, on ajoute 80 g d'alumine à 100 m²/g; le mélange est maintenu sous agitation durant environ 12 h. Après élimination de l'eau sous vide, on maintient environ 12 h à 140°C sous 130 pascals de pression résiduelle; puis, on chauffe la poudre ainsi obtenue à 270°C sous courant d'azote.

On place 8 g du catalyseur ainsi obtenu dans un tube réactionnel de 0,95 cm de diamètre et on le maintient à 270°C, toujours sous courant d'azote. On fait passer ensuite sur ce lit catalytique un mélange de $CH_3Cl$ et $Cl_2$ dans un rapport molaire de 1 : 2 avec une vitesse spatiale de 240, définie à température ambiante par le volume de gaz en ml par g de catalyseur et par heure. Des prélèvements gazeux effectués après 1 à 2 heures donnent une composition sensiblement constante du mélange gazeux réactionnel exprimé en pourcentages pondéraux:

| | |
|---|---|
| $CH_3Cl$ | 57% |
| $CH_2Cl_2$ | 37,8% |
| $CHCl_3$ | 5,2% |
| $CCl_4$ | 0% |

soit une sélectivité en $CH_2Cl_2$ de 88% et un taux de conversion du chlorure de méthyle de 43%.

### Exemple 2

Cet exemple illustre la transformation du chlorure de méthyle en chlorure de méthylène, en utilisant un catalyseur à base de platine déposé sur alumine à raison de 0,5% pt pondéral par rapport à Al₂O₃.

En opérant dans l'appareillage de l'exemple 1, on introduit 10 g de catalyseur dans le tube réactionnel qu'on conditionne quelques heures sous courant d'azote sec. Puis, on fait passer sur le catalyseur un mélange gazeux CH₃Cl et Cl₂ en volume égaux, avec une vitesse spatiale de 360 telle que définie dans l'exemple 1. Dans ces conditions, le mélange réactionnel sortant a pour composition pondérale:

| | |
|---|---|
| CH₃Cl | 60% |
| CH₂Cl₂ | 32,8% |
| CHCl₃ | 7,2% |
| CCl₄ | traces |

soit une sélectivité en CH₂Cl₂ de 82% et un taux de conversion du CH₃Cl de 40%.

### Exemple 3

A une solution agitée de 13,4 g de ZrCl₄ dans 120 ml d'eau, on ajoute 200 g d'alumine à 250 m²/g. Le mélange est maintenu sous agitation pendant 1 heure. Après élimination de l'eau sous vide, on maintient 24 h à 90°C sous une pression résiduelle de 1300 pascals.

Le catalyseur est ensuite placé dans un tube réactionnel de 4 cm de diamètre où il est maintenu à 190°C sous courant d'azote. On fait passer ensuite sur le catalyseur un mélange de CH₃Cl et Cl₂ dans un rapport 1—3 avec une vitesse spatiale de 78 telle que définie dans l'exemple 1.

Dans ces conditions, la composition pondérale du mélange réactionnel sortant est la suivante:

| | |
|---|---|
| CH₃Cl | 50,2% |
| CH₂Cl₂ | 41,8% |
| CHCl₃ | 7,5% |
| CCl₄ | 0,6% |

soit une sélectivité en CH₂Cl₂ de 83,8% et un taux de conversion de CH₃Cl de 49,8%

### Revendications

1. Procédé de fabrication du chlorure de méthylène par chloration ionique ou initiation par un métal noble du chlorure de méthyle caractérisé en ce qu'on fait réagir un mélange de chlorure de méthyle et de chlore dans un rapport molaire compris entre 3 et 0,25 en présence d'un catalyseur à base d'un métal choisi dans les groupes III, IV, V, VI, VIII de la table périodique.

2. Procédé selon la revendication 1 caractérisé en ce que la chloration s'effectue à une température comprise entre 150 et 350°C.

3. Procédé selon la revendication 1 caractérisé en ce que la chloration s'effectue à une température comprise entre 170 et 300°C.

4. Procédé selon l'une des revendications 1 à 2 caractérisé en ce que le catalyseur se trouve sous forme métallique, d'halogénure ou d'oxyhalogénure.

### Patentansprüche

1. Verfahren zur Herstellung von Methylenchlorid durch ionische Chlorierung oder mit einem Edelmetall initiierte Chlorierung von Methylchlorid, dadurch gekennzeichnet, daß man ein Gemisch aus Methylchlorid und Chlor in einem Molverhältnis von 3 bis 0,25 in Gegenwart eines Katalysators auf der Basis eines Metalls, ausgewählt aus den Gruppen III, IV, V, VI und VIII des Periodensystems, reagieren läßt.

2. Verfahren nach Anspruch 1, dadurch gekennzeichnet, daß die Chlorierung bei einer Temperatur zwischen 150 und 350°C erfolgt.

3. Verfahren nach Anspruch 1, dadurch gekennzeichnet, daß die Chlorierung bei einer Temperatur zwischen 170 und 300°C erfolgt.

4. Verfahren nach einem der Ansprüche 1 bis 2, dadurch gekennzeichnet, daß der Katalysator in metallischer Form oder in Form eines Halogenids oder Oxyhalogenids vorliegt.

### Claims

1. Process for the manufacture of methylene chloride by ionic chlorination or initiation of methyl chloride with a noble metal, characterised in that a mixture of methyl chloride and chlorine, in a molar ratio of between 3 and 0.25, is reacted in the presence of a catalyst based on a metal chosen from groups III, IV, V, VI and VIII of the periodic table.

2. Process according to Claim 1, characterised in that the chlorination is carried out at a temperature of between 150 and 350°C.

3. Process according to Claim 1, characterised in that the chlorination is carried out at a temperature of between 170 and 300°C.

4. Process according to one of Claims 1 to 2, characterised in that the catalyst is in the form of a metal, halide or oxyhalide.